# EUROPEAN PATENT APPLICATION

(11) **EP 4 406 753 A1**
(43) Date of publication of application: **31.07.2024**
(21) Application number: 24153340.5
(22) Date of filing: 23.01.2024
(51) Int. Cl.: B44C 1/22, G01N 21/87, H04L 9/08, H04L 9/32, B28D 5/00, G01N 33/00

(54) **METHOD AND SYSTEM FOR DETERMINING AUTHENTICITY OF A MANUFACTURED DIAMOND**

(30) Priority: 26.01.2023 CH 862023
(71) Applicant: Frank Ziemer Holding AG, 2562 Port (CH)
(72) Inventor: Ziemer, Frank, 2562 Port (CH); Chodelka, Robert, 2562 Port (CH); Rathjen, Christian, 28197 Bremen (DE); Steinlechner, Michael, 8006 Zürich (CH)
(74) Representative: Rentsch Partner AG

(57) **Abstract**

A computerized system (100) for determining the authenticity of a diamond (1) comprises a computerized certification system 2 and a computerized authentication system 3. The computerized certification system 2 is configured to certify a diamond (1) by generating (S11) diamond reference characteristics, using one or more physical characteristics of the diamond (1) measured during a certification phase, and writing a visual code in or on the diamond (1), using a beam writing system. The computerized authentication system (3) is configured to authenticate the diamond (1) by generating (S21) diamond verification measurements, using one or more physical characteristics of the diamond (1), measured during an authentication phase, capturing the visual code of the diamond (1), and comparing the diamond verification measurements and the diamond reference characteristics, using the visual code captured from the diamond (1).

## Description

### FIELD OF THE DISCLOSURE

The present disclosure relates to a method and a system for determining authenticity of a manufactured diamond. Specifically, the present disclosure relates to a method of determining authenticity of a manufactured diamond and a computerized authentication system for verifying the authenticity of the manufactured diamond.

### BACKGROUND OF THE DISCLOSURE

Manufactured diamonds, also referred to as laboratory-grown diamonds or artificial, synthetic, or cultured diamonds, are produced in a controlled technological process. Manufactured diamonds are composed of the same element as naturally formed diamonds, pure carbon crystallized in an isotropic 3D form, and share identical chemical and physical properties. The most prominent processes include the high-pressure high-temperature method (HPHT) and the chemical vapor deposition method (CVD). For example, the HPHT diamonds are manufactured at temperatures around 1500 °C (2732.0 °F) and pressures around 55,000 bar (850,000 psi) to replicate the natural geological diamond growth process that occurs deep within the earth. Particularly for valuable and/or unique jewelry grade diamonds, it would be advantageous to be able to reliably authenticate a specific, individual diamond. For example, for the purpose of linking the particular diamond to its manufacturer, its production date, its production time, its production location, its source(s) of carbon material, its owner, and/or its provenance, i.e. its chronological history of ownership. A reliable authentication of a specific, individual synthetic diamond would also make it possible to detect falsifications or imitations of this particular synthetic diamond.

US 2021/158118 A1 discloses a secured product identification method for products such as precious gemstones to prevent counterfeiting by incorporating a marked product with a fake identification (ID) mark or fake barcode. According to US 2021/158118 A1, the authentication process requires at least two identifying data sets, an overt mark or overt data and covert data. The overt data may be a unique product identifier like a barcode. Covert data may be any additional data derived from the specific product. Additional data points come from a created data point not originally part of the product, or a unique data point already existing in the product but not existing in any other similar product. The product is authenticated with centralized or de-centralized databases when the combination of data sets compared to the original data sets is positive. A partially or non-automated method may be implemented, for example, where a barcode links to the database. Authentication relying on centralized or de-centralized databases is impaired when these databases cannot be accessed, e.g. because of lack of network connectivity or network problems, or the databases are not available, e.g. because of maintenance downtime.

US 7,655,882 B2 discloses an apparatus and method for producing an authentication certification for a gemstone, having a processor, a database coupled to the processor, in which are stored data defining laser micro-inscriptions and physical characteristics of a plurality of gemstones. A graphic user interface (GUI) presents in human readable form information from the database describing for a respective gemstone the laser micro-inscription and physical characteristic information. The output is used for authentication of a presumptive gemstone. In an embodiment, instead of comparison with metric data stored in the database, the laser marking inscribed on the gemstone includes an encrypted message containing data relating to characteristics of the stone. A so-called public key/private key encryption protocol is used to label the gemstone with a "digital signature". The encoding party codes the data using a private key. The message is decoded using the associated public key. The data in the deciphered message includes a set of unique or quasi unique characteristics of the gemstone. Thereby, to verify the origin of the gemstone and its authenticity, the information from the decoded message is compared with the gemstone. While this "self-authenticating" embodiment makes it possible to verify the gemstone without access to a database, it also makes it possible for a malicious party to copy the gemstone by deciphering the characteristics of the gemstone, using the public key, and to manufacture a gemstone with the same characteristics, including a copy of the laser marking inscribed on the original gemstone.

### SUMMARY OF THE DISCLOSURE

It is an object of this disclosure to provide a method of determining authenticity of a manufactured diamond and a computerized authentication system for verifying the authenticity of the manufactured diamond.

According to the present disclosure, these objects are addressed by the features of the independent claims. In addition, further advantageous embodiments follow from the dependent claims and the description.

According to the present disclosure, the above-mentioned objects are particularly achieved in that for determining the authenticity of a manufactured diamond, a computerized certification system generates diamond reference characteristics by capturing measurements of one or more physical characteristics of the diamond during a certification phase. The computerized certification system generates a hash of the diamond reference characteristics by applying a cryptographic hash function to the diamond reference characteristics. The computerized certification system generates a visual code for the diamond, using the hash of the diamond reference characteristics. Using a beam writing system, the visual code is written in or on the diamond. A computerized authentication system verifies the authenticity of the diamond by generating diamond verification measurements by capturing measurements of the one or more physical characteristics of the diamond during an authentication phase. The computerized authentication system generates a hash of the diamond verification measurements by applying the cryptographic hash function to the diamond verification measurements. The computerized authentication system captures the visual code of the diamond and compares the hash of the diamond verification measurements with the hash of the diamond reference characteristic, using the visual code captured from the diamond. Depending on the comparing, the computerized authentication system affirms or denies the authenticity of the diamond. For example, the beam writing system is a laser beam writing system, an ion beam writing system, or an electron beam writing system.

It should be noted that the term "visual code" refers to an optically detectable code. Accordingly, a visual code reader is configured to optically detect said code.

In an embodiment, during the certification phase, generating the hash of the diamond reference characteristics ("reference hash") comprises a discretization (or rounding) of the diamond reference characteristics of the diamond prior to applying the cryptographic function, specifically, the cryptographic hash function. Likewise, during the authentication phase, generating the hash of the diamond verification measurements ("verification hash") comprises a discretization (or rounding) of the diamond verification measurements of the diamond prior to applying the cryptographic function, specifically, the cryptographic hash function.

In an embodiment, the computerized certification system stores the diamond reference characteristics assigned to a unique diamond identifier in a data storage system; the computerized certification system includes the unique diamond identifier in the visual code; the computerized authentication system determines the diamond identifier from the visual code; and the computerized authentication system compares the diamond verification measurements to at least some of the diamond reference characteristics, stored and assigned in the data storage system to the diamond identifier.

In an embodiment, comparing the diamond verification measurements with the diamond reference characteristics comprises retrieving at least some of the diamond reference characteristics via a network from the data storage system, and/or transmitting at least some of the diamond verification measurements and the diamond identifier via the network to a verification server.

In an embodiment, generating the visual code comprises including in the visual code addressing information of the data storage system and/or the verification server; and comparing the diamond verification measurements with the diamond reference characteristics comprises using the addressing information included in the visual code for retrieving at least some of the diamond reference characteristics from the data storage system and/or transmitting at least some of the diamond verification measurements and the diamond identifier to the verification server.

In an embodiment, the computerized certification system stores the hash of the diamond reference characteristics in a Blockchain data storage system; and the computerized authentication system compares the hash of the diamond verification measurements with the hash of the diamond reference characteristics stored in the Blockchain data storage system.

In an embodiment, markings are generated in or on the diamond, using a beam writing system. For example, the beam writing system is laser beam writing system, an ion beam writing system, or an electron beam writing system.

In various embodiments, generating the markings in or on the diamond comprises varying an energy level of the beam writing system, varying a degree of overlap of pulses of the beam writing system, varying a projection direction of a beam of the beam writing system, and/or varying a focal position of the beam of the beam writing system.

In an embodiment, markings are generated in or on the diamond, using potassium nitrate, plasma etching in combination with masking materials, gas phase etching in dry oxygen, gas phase etching in a mixture of oxygen and water vapor, and/or liquid etching in molten potassium nitrate. For example, the masking material or the mask(s), respectively, is/are applied by way of printing on the surface of the diamond.

In various embodiments, measuring the one or more physical characteristics of the diamond comprises measuring a weight of the diamond, measuring a three-dimensional geometric shape of the diamond, measuring a color spectrum of the diamond, measuring a chemical composition of the diamond, measuring markings in or on the diamond, measuring inclusions in the diamond, and/or measuring a hologram in the diamond.

The physical characteristics of the diamond, used as diamond reference characteristics, and the diamond verification measurements include one or more physical characteristics of the diamond which are deterministic characteristics of the diamond. Depending on the embodiment, the deterministic physical characteristics of the diamond used as diamond reference characteristics and diamond verification measurements include, the weight of the diamond, the three-dimensional geometric shape of the diamond, the color spectrum of the diamond, the chemical composition of the diamond, markings in or on the diamond, and/or inclusions in the diamond. The markings in or on the diamond and/or inclusions in the diamond are represented by relative location data and/or dimension data descriptive of the markings and/or inclusions. In an embodiment, the markings and/or inclusions are defined by a marking map, a three-dimensional marking model, an inclusion map, and/or a three-dimensional inclusion model.

In an embodiment, the deterministic physical characteristics of the diamond used as diamond reference characteristics and diamond verification measurements further include refraction index and conductance value of the diamond. This enables distinction of the diamond from other materials.

In an embodiment, generating the diamond reference characteristics comprises the computerized certification system including a secret diamond code in the diamond reference characteristics; and generating the verification measurements comprises the computerized authentication system receiving from a user the secret diamond code and including the secret diamond code received from the user in the diamond verification measurements.

In an embodiment, generating the visual code comprises generating a QR-code (Quick Response), and writing the visual code in or on the diamond comprises writing the QR-code in or on the diamond using the beam writing system.

In an embodiment, affirming the authenticity of the diamond comprises the computerized authentication system generating an authentication message. In an embodiment, the authentication message includes a digital signature, which is verifiable by a trusted third party.

In an embodiment, generating the visual code comprises the computerized certification system including in the visual code addressing information of the trusted third part.

In an embodiment, generating the visual code comprises the computerized certification system including in the visual code addressing information of the data storage system.

In an embodiment, generating the visual code comprises the computerized certification system including in the visual code Blockchain addressing information.

In addition to the method for determining the authenticity of a manufactured diamond, the present disclosure also relates to a computerized authentication system for verifying the authenticity of a manufactured diamond, which comprises a visual code. The computerized authentication system comprises a processing unit and a sensor system connected to the processing unit. The processing unit is configured to capture one or more physical characteristics of the diamond measured by the sensor system during an authentication phase, and to generate diamond verification measurements using the one or more physical characteristics of the diamond measured during the authentication phase. The processing unit is further configured to generate a hash of the diamond verification measurements, by applying a cryptographic hash function to the diamond verification measurements, and to capture the visual code of the diamond. Using the visual code captured from the diamond, the processing unit is further configured to compare the hash of the diamond verification measurements with a hash of diamond reference characteristic, generated by a computerized certification system from one or more physical characteristics of the diamond during a certification phase, and to affirm or deny the authenticity of the diamond depending on the comparison.

In addition to the method and system for determining the authenticity of a manufactured diamond, the present disclosure also relates to a computer program product comprising computer program code for controlling a processing unit of a computerized authentication system to verify the authenticity of a manufactured diamond which comprises a visual code. In particular, the present disclosure relates to a computer program product comprising a computer readable medium having stored thereon the computer program code. More particularly, the present disclosure relates to a computer program product comprising a non-transitory computer readable medium having stored thereon the computer program code. The computer program code is configured to control the processing unit of the computerized authentication system to verify the authenticity of the manufactured diamond by performing the following steps: capturing one or more physical characteristics of the diamond measured by a sensor system of the computerized authentication system during an authentication phase; generating diamond verification measurements using the one or more physical characteristics of the diamond measured during the authentication phase; generating a hash of the diamond verification measurements by applying a cryptographic hash function to the diamond verification measurements; capturing the visual code of the diamond; using the visual code captured from the diamond to compare the hash of the diamond verification measurements with a hash of diamond reference characteristic, generated by a computerized certification system from one or more physical characteristics of the diamond during a certification phase; and affirming or denying the authenticity of the diamond depending on the comparison.

### BRIEF DESCRIPTION OF THE DRAWINGS

The present disclosure will be explained in more detail, by way of example, with reference to the drawings in which:
- Figure 1: shows a block diagram illustrating schematically a system for determining authenticity of a manufactured diamond, the system comprising a computerized certification system and a computerized authentication system.
- Figure 2: shows a block diagram illustrating schematically a computerized certification system comprising a processing unit, a sensor system, a beam writing system, and a data storage system.
- Figure 3: shows a block diagram illustrating schematically a computerized certification system comprising a processing unit, a sensor system, and a beam writing system.
- Figure 4: shows a block diagram illustrating schematically a computerized authentication system which comprises a sensor system and a processing unit, connected via a network to a data storage system.
- Figure 5: shows a block diagram illustrating schematically a computerized authentication system which comprises a sensor system, a processing unit, and a verification server which has a data storage system and is connected to the processing unit via a network.
- Figure 6: shows a block diagram illustrating schematically a computerized authentication system which comprises a sensor system and a processing unit.
- Figure 7: shows a block diagram illustrating schematically a computerized authentication system which is implemented as a mobile device and comprises a sensor system and a processing unit, the mobile device being optionally connected via a network to a data storage system.
- Figure 8: shows a flow diagram illustrating an exemplary sequence of steps for determining authenticity of a diamond, including certifying the diamond in a certification phase and verifying authenticity of the diamond in an authentication phase.
- Figure 9: shows a flow diagram illustrating an exemplary sequence of steps for certifying a diamond by generating diamond reference characteristics of a diamond during a certification phase, whereby diamond reference characteristics are determined prior to writing a visual code in or on the diamond.
- Figure 10: shows a flow diagram illustrating an exemplary sequence of steps for certifying a diamond by generating diamond reference characteristics of a diamond during a certification phase, whereby a visual code is written in or on the diamond prior to determining diamond reference characteristics.
- Figure 11: shows a flow diagram illustrating an exemplary sequence of steps for certifying a diamond by generating diamond reference characteristics of a diamond during a certification phase, whereby diamond reference characteristics are determined prior to writing a visual code in or on the diamond and further diamond reference characteristics are determined after the visual code is written in or on the diamond.
- Figure 12: shows a flow diagram illustrating an exemplary sequence of steps for authenticating a diamond during an authentication phase, including determining diamond verification measurements, capturing a visual code of the diamond, and assessing correspondence of the diamond verification measurements with diamond reference characteristics of the diamond.
- Figure 13: shows a flow diagram illustrating an exemplary sequence of steps for assessing correspondence of diamond verification measurements with diamond reference characteristics, including determining a diamond identifier from a visual code and retrieving the diamond reference characteristics from a data storage system.
- Figure 14: shows a flow diagram illustrating an exemplary sequence of steps for assessing correspondence of diamond verification measurements with diamond reference characteristics, including determining a diamond identifier from a visual code and transmitting the diamond verification measurements to a verification server.
- Figure 15: shows a flow diagram illustrating an exemplary sequence of steps for assessing correspondence of diamond verification measurements with diamond reference characteristics, including determining diamond reference characteristics from a visual code.
- Figure 16: shows a flow diagram illustrating an exemplary sequence of steps for assessing correspondence of diamond verification measurements with diamond reference characteristics, including generating transformed diamond verification measurements and determining transformed diamond reference characteristics from a visual code.
- Figure 17: shows a flow diagram illustrating an exemplary sequence of steps for assessing correspondence of diamond verification measurements with diamond reference characteristics, including generating transformed diamond verification measurements, determining Blockchain addressing information from a visual code, and determining transformed diamond reference characteristics in a Blockchain.
- Figure 18: shows a flow diagram illustrating an exemplary sequence of steps for assessing correspondence of diamond verification measurements with diamond reference characteristics, including generating transformed diamond verification measurements, determining a Blockchain account address from a visual code, and submitting the transformed diamond verification measurements to the Blockchain account.

### DETAILED DESCRIPTION OF THE EMBODIMENTS

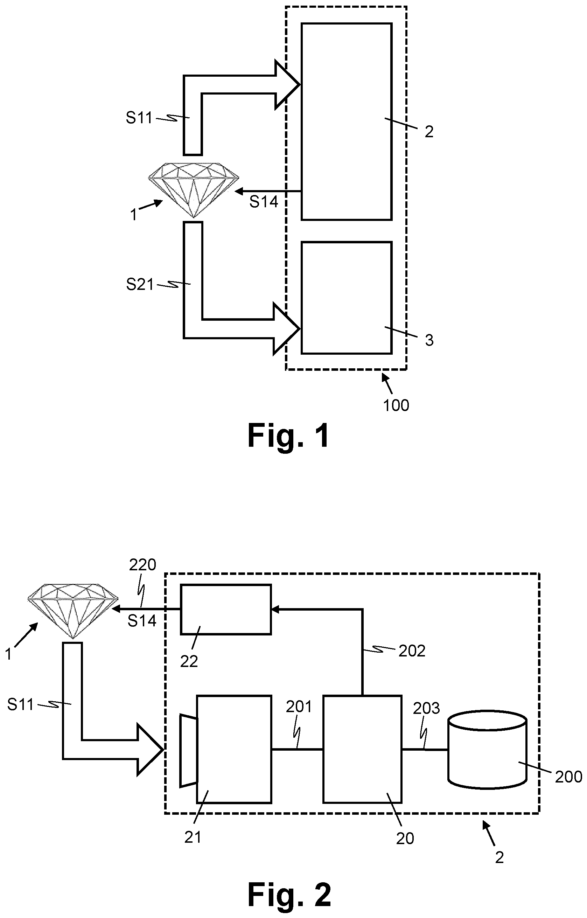

In Figure 1, reference numeral 100 refers to a computerized system for determining the authenticity of a diamond 1, particularly a manufactured diamond 1. As illustrated in Figure 1, the system 100 comprises a computerized certification system 2 and a computerized authentication system 3. The computerized certification system 2 is configured to certify a diamond 1 in a certification phase. The computerized authentication system 3 is configured to authenticate a diamond 1 in an authentication phase.

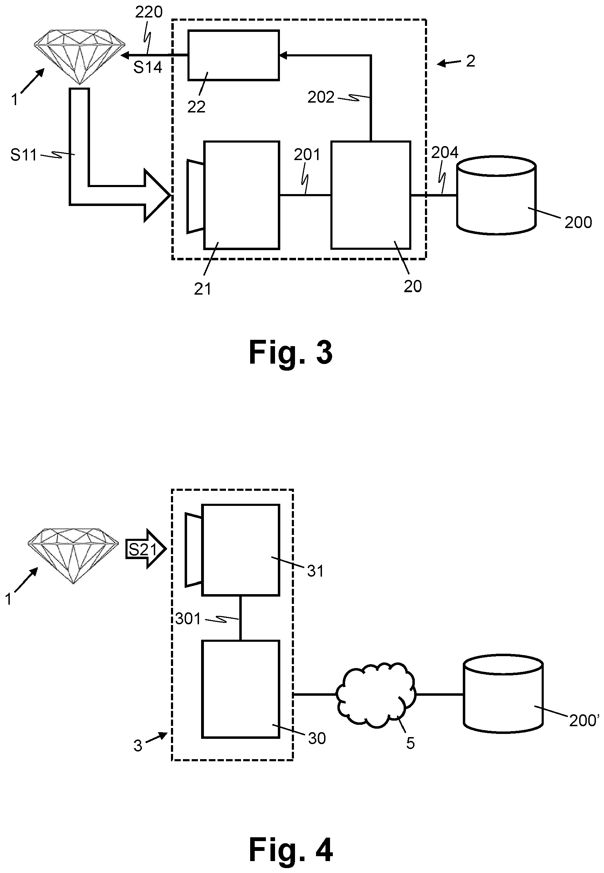

As illustrated in Figures 2 and 3, the computerized certification system 2 comprises a processing unit 20, a sensor system 21, and a beam writing system 22. For example, the beam writing system 22 is laser beam writing system, an ion beam writing system, or an electron beam writing system. The processing unit 20 is connected to the sensor system 21 and the beam writing system 22 via wired or wireless communication links or networks 201, 202. Alternatively, data communication between the processing unit 20 and the sensor system 21 and/or between the processing unit 20 and the beam writing system 22 is accomplished by a human user via user interfaces and/or a transferable data storage medium, such as a flash memory module.

In Figures 2 and 3, reference numeral 200 refers to a data storage system. Depending on the embodiment, the data storage system 200 comprises data storage memory, data storage disks, and one or more computers for data storage management, database management, and/or distributed ledger systems, such as Blockchain systems. In the embodiment of Figure 2, the data storage system 200 is part of the computerized certification system 2, connected to the processing unit 20 via a wired or wireless communication link or network 203.

The wired or wireless communication links or networks 201, 202, 203 include communication busses, LANs (Local Area Network), or WLANs (Wireless Local Area Network) or other wireless communication links, such as Bluetooth or RFID (Radio Frequency Identifier).

In the embodiment of Figure 3, the data storage system 200 is arranged remotely from the computerized certification system 2, connected to the computerized certification system 2 and its processing unit via a telecommunication network 204, including a mobile radio network, such as a GSM (Global System for Mobile Communication) or UMTS (Universal Mobile Telephone System) radio network, and/or the Internet.

The processing unit 20 comprises a computer, a processor, and/or an electronic circuit configured to control the sensor system 21 and/or the beam writing system 22, and to communicate data with the sensor system 21 and/or the beam writing system 22, and the data storage system 200, as will be described later in more detail.

The beam writing system 22 is configured to write a visual code, i.e. an optically detectable code, e.g. a QR code (Quick Response), a bar code, and/or an alphanumeric code in or on the diamond 1. The beam writing system 22 comprises a beam source, e.g. a laser source, configured to generate a laser beam, e.g. a laser source for generating a pulsed laser beam, such as a nano, pico or femto second laser. Alternatively, the beam writing system 22 comprises a beam source configured to generate an ion beam or an electron beam. The beam writing system 22 further comprises a scanner system and a focusing system configured to direct and focus the beam to write the visual code in or on the diamond 1.

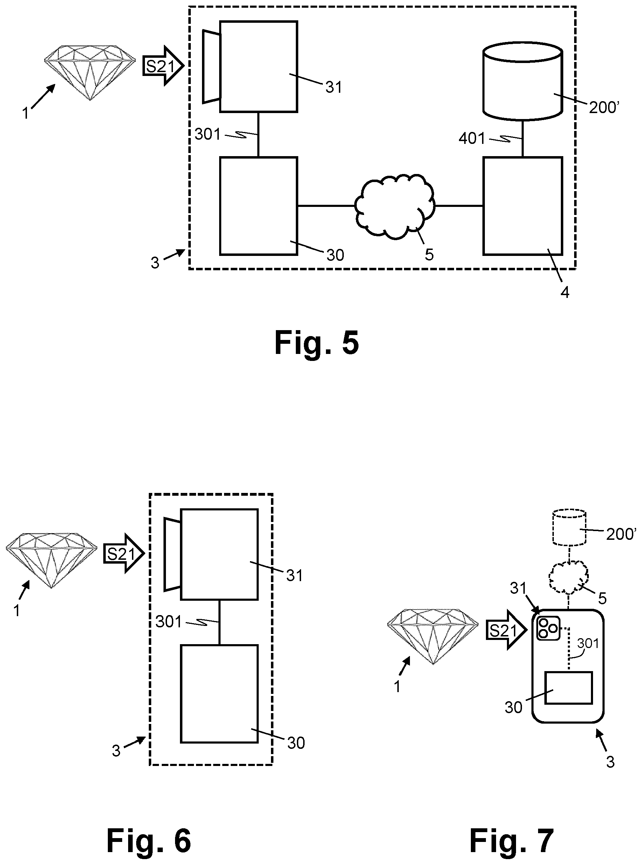

As illustrated in Figures 4-7, the computerized authentication system 3 comprises a processing unit 30 and a sensor system 31. The processing unit 30 and the sensor system 31 are separate devices with their own housings or integrated in one device with one housing. Figure 7 illustrates an example of a device that comprises both the processing unit 30 and the sensor system 31. For example, the device of Figure 7 is implemented as a mobile device, e.g. a smart phone, a tablet or a notebook computer.

In Figures 4, 5 and 7, reference numeral 200' refers to a data storage system. Depending on the embodiment, the data storage system 200' shown in Figures 4, 5 and 7 corresponds to (i.e. is the same as) the data storage system 200 discussed above and shown in Figures 2 and 3, or the data storage system 200' shown in Figures 4, 5 and 7 is a separate data storage system with the same or a subset of the data content of the data storage system 200, discussed above and shown in Figures 2 and 3.

In the embodiment of Figure 4, and optionally in the embodiment of Figure 7, the computerized authentication system 3 or its the processing unit 30, respectively, is connected with the data storage system 200' via a telecommunication network 5, including a mobile radio network, such as a GSM (Global System for Mobile Communication) or UMTS (Universal Mobile Telephone System) radio network, and/or the Internet.

In the embodiment of Figure 5, the computerized authentication system 3 further comprises a verification server 4 connected to the processing unit 30 via telecommunication network 5. The verification server 4 comprises one or more computers, with one or more processors, connected to the data storage system 200' via communication link or network 401, including communication busses, LANs (Local Area Network), or WLANs (Wireless Local Area Network).

As illustrated in Figures 4-7, the processing unit 30 is connected to the sensor system 31 via wired or wireless communication link or network 301. Depending on the embodiment, the wired or wireless communication link or network 301 includes optical interfaces, communication busses, LANs, or WLANs or other wireless communication links, such as Bluetooth or RFID. Alternatively, data communication between the processing unit 30 and the sensor system 31 is accomplished by a human user via user interfaces and/or a transferable data storage medium, such as a flash memory module.

The processing unit 30 comprises a computer, a processor, and/or an electronic circuit configured to communicate data with the data storage system 200', as will be described later in more detail. Depending on the embodiment and/or configuration, the computer, processor, and/or electronic circuit of the processing unit 30 is further configured to control the sensor system 31, and to communicate data with the sensor system 31.The sensor systems 21, 31 comprise one or more devices with one or more sensors configured to measure physical characteristics of the diamond 1. For example, the sensor systems 21, 31 comprise a scale for measuring the weight of the diamond 1, a spectrometer for measuring the color spectrum of the diamond 1, a mass spectrometer for measuring the chemical composition of the diamond 1, one or more visual sensors, e.g. cameras, for capturing images of the diamond 1, markings in or on the diamond 1, and/or inclusions in the diamond 1, and/or depth or distance sensors for measuring three-dimensional topographies of the diamond 1, one or more tomographic systems, such as an optical coherence tomography system (OCT) or a confocal microscope, for capturing tomographic data of inclusions and/or markings and their locations in the diamond 1, and/or a combined laser and camera system configured to read a hologram generated by a laser beam of the laser directed on and reflected by structures in the diamond 1. The sensor systems 21, 31 further comprise microscopes optically linked to cameras for capturing magnified images of the diamond 1, markings in or on the diamond 1, and/or inclusions in the diamond 1. In an embodiment, the sensor systems 21, 31 further comprise measurement devices, e.g. a refractometer and/or an impedance meter, for measuring the refraction index and/or the conductance of the diamond 1. The sensor systems 31 of the computerized authentication system 3 comprises a visual code reader configured to read the visual code off the diamond 1. Depending on the embodiment, the visual code reader comprises a camera and/or a scanner, for example linked optically with an optical magnifying system, such as a microscope or a magnifying lens, configured to magnify the visual code written in or on the diamond 1.

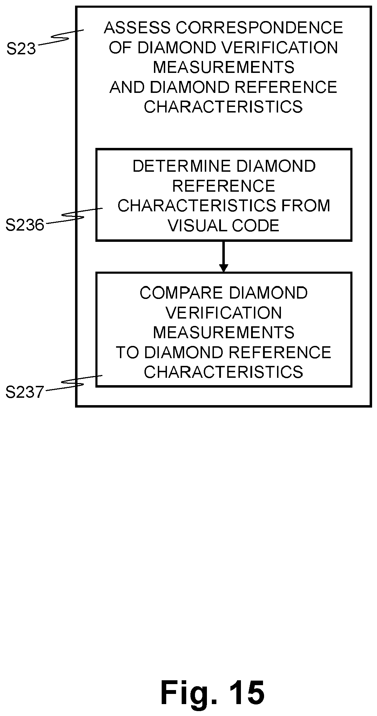

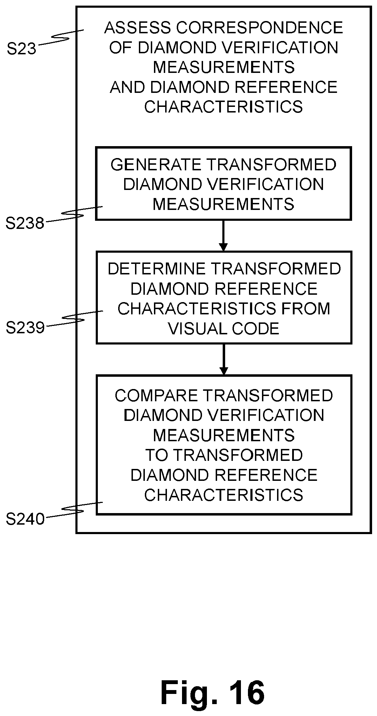

The embodiments or configurations of the computerized authentication system 3 shown in Figures 6 and 7 (without optional access to the data storage system 200') are particularly suitable for off-line verification of the diamond 1, where assessing the correspondence of the diamond verification measurements and the diamond reference characteristics of the diamond 1 does not require access to the data storage system 200' or the verification server 4, as will be described later with reference to Figures 15 and 16.

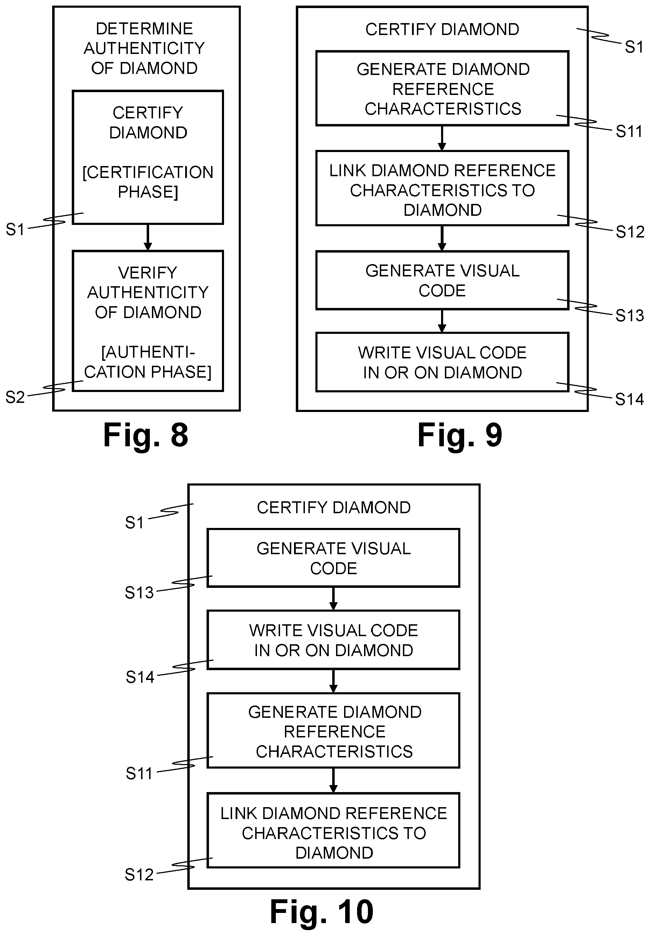

In the following paragraphs, described with reference to Figures 8-18 are possible sequences of steps performed by the computerized certification system 2 or its processing unit 20 for certifying a diamond 1 in step S1 during a certification phase, and by the computerized authentication system 3 for authenticating the diamond 1 in step S2 during an authentication phase (see Figure 8).

As illustrated in Figure 9, for certifying the diamond 1 in step S1 during the certification phase, in step S11, the computerized certification system 2 generates diamond reference characteristics of the diamond 1. More specifically, the processing unit 20 of the computerized certification system 2 controls the sensor system 21 of the computerized certification system 2 to measure one or more physical characteristics of the diamond 1. The processing unit 20 of the computerized certification system 2 generates the diamond reference characteristics of the diamond 1 by forming a data record or data set including the measured physical characteristics of the diamond 1. The measured physical characteristics of the diamond 1 constitute deterministic characteristics of the diamond 1. As described above, examples of the measured physical characteristics of the diamond 1 include the weight of the diamond 1, the color spectrum of the diamond 1, the chemical composition of the diamond 1, one or more images of the diamond 1, measurements and/or images of markings in or on the diamond 1, measurements and/or images of inclusions in the diamond 1, three-dimensional topographies of the diamond 1, tomographic data of inclusions and/or markings and their locations in the diamond 1, and/or a hologram. In an embodiment, the measured physical characteristics of the diamond 1 further include the refraction index and/or the conductance of the diamond 1.

In an embodiment, in step S11, the processing unit 20 of the computerized certification system 2 further generates and includes in the diamond reference characteristics of the diamond 1 a secret diamond code, e.g. a code comprising several digits, e.g. 10, 20, or more alphanumeric and/or special character digits (e.g. "+", "*", "&", etc.).

In an embodiment, in step S11, the processing unit 20 of the computerized certification system 2 further generates markings in or on the diamond, prior to measuring the one or more physical characteristics of the diamond 1. More specifically, the processing unit 20 of the computerized certification system 2 controls a beam writing system 22 to generate the markings in or on the diamond. For example, the markings are generated with varying energy level of the beam writing system 22, varying the degree of overlap of pulses of the beam writing system 22, varying the projection direction of the beam of the beam writing system 22, and/or varying the focal position of the beam of the beam writing system 22. In an embodiment, in addition or alternatively, markings are generated on the diamond, using potassium nitrate, plasma etching in combination with masking materials, gas phase etching in dry oxygen, gas phase etching in a mixture of oxygen and water vapor, and/or liquid etching in molten potassium nitrate. For example, the masking material or the mask(s), respectively, is(are) applied by way of printing on the surface of the diamond.

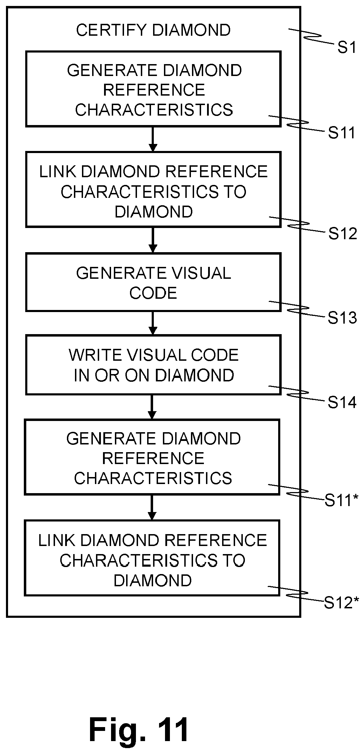

The measurements of markings in or on the diamond 1 include shape, dimensions, as well as position and orientation in or on the diamond 1. The same applies to measurements of the visual code as a marking, in cases where the measurements are taken after the visual code is written in or on the diamond 1, as will described later with reference to Figures 10 and 11.

In step S12, the computerized certification system 2 or its processing unit 20, respectively, links the diamond reference characteristics to the diamond 1. More specifically, the processing unit 20 of the computerized certification system 2 generates a unique identifier for the diamond 1 and stores the diamond reference characteristics assigned to the unique diamond identifier in the data storage system 200. Alternatively or in addition, the processing unit 20 of the computerized certification system 2 generates transformed diamond reference characteristics by applying a cryptographic function to the diamond reference characteristics, particularly a cryptographic hash function, and stores the transformed diamond reference characteristics, particularly the cryptographic hash value of the diamond reference characteristics ("reference hash"), assigned to the unique diamond identifier in a Blockchain data storage system 200, e.g. an Ethereum Blockchain. A cryptographic hash function is related to a one-way function and generates a digital fingerprint from an input, such as a data set or message. The cryptographic hash function generates from the input *m* a hash value *h* in such a way that for a given hash value *h,* it is difficult to find any input *m* such that *h* = hash(*m*), whereby "difficult" refers to computational complexity. Furthermore, given an input *m₁*, it is difficult to find a different input *m₂* such that hash(*m₁*) = hash(*m₂*). Examples of cryptographic hash functions include Message Digest 5 (MD5); the Secure Hash Algorithms (SHA), a family of cryptographic hash functions published by the National Institute of Standards and Technology (NIST), including SHA-1, SHA-2, and SHA-3; and BLAKE2 or BLAKE3. The unique diamond identifier includes a serial number, a manufacturing number, a manufacturing date, a manufacturing time, and/or a material reference code. Depending on the embodiment or configuration, the material reference code is linked in the data storage system 200 to source information about the specific source of at least some of the material used for generating the diamond 1. The source information may include a container reference of a container which holds some sample of the original material used for producing the diamond 1, multimedia information, such as video, images, and/or audio recordings, related to the source and/or material used for producing the diamond 1, and/or addressing information, such as URL or other linking information, directing to such multimedia information on the Internet.

In an embodiment, during the certification phase, generating the transformed diamond reference characteristics ("reference hash") comprises a discretization of the diamond reference characteristics of the diamond 1 prior to applying the cryptographic function, specifically, the cryptographic hash function. In other words, the various diamond reference characteristics are discretized or rounded in that they are represented by discrete or "granular" values, with a defined accuracy or resolution of the specific physical characteristics. Likewise, during the authentication phase, generating transformed diamond verification measurements ("verification hash") comprises a discretization of the diamond verification measurements of the diamond 1 prior to applying the cryptographic function, specifically, the cryptographic hash function. By discretizing or rounding the physical characteristics of the diamond 1, the various physical characteristics of the diamond 1 are input to the cryptographic function, specifically, the cryptographic hash function, with their respective accuracy and precision. This makes it possible, to account for different accuracies and precision of measuring the physical characteristics of the diamond 1 as diamond reference characteristics, in the certification phase, and as diamond verification measurements, in the authentication phase. For example, prior to applying the cryptographic function, specifically, the cryptographic hash function, measures of distance of the diamond 1 are discretized or rounded to a precision of 10 µm, preferably 5 µm, and measures of weight of the diamond 1 are rounded to a precision of 10 mg, preferably 5 mg. Consequently, this amounts to tolerance thresholds smaller than 10 µm, preferably smaller than 5 µm, for measures of distance, and tolerance thresholds smaller than 10 mg, preferably smaller than 5 mg, for measures of weight. This makes it possible for a jeweler, for example, to use measurement equipment with a lower accuracy or precision for determining the verification measurements during authentication phase, than the measurement equipment used to measure the diamond reference characteristics during the certification phase.

In step S13, the computerized certification system 2 or its processing unit 20, respectively, generates a visual code for the diamond 1, e.g. a QR code or a bar code. In an embodiment, the visual code includes the unique diamond identifier. Alternatively or in addition, the visual code includes addressing information of a trusted third party (e.g. a PKI trusted third party), of the verification server 4, and/or of the data storage system 200, 200', e.g. a hyperlink or other uniform resource locator (URL), and/or Blockchain addressing information such as a Blockchain account address or a Blockchain transaction identifier of a Blockchain record. Alternatively or in addition, the computerized certification system 2 or its processing unit 20, respectively, uses the diamond reference characteristics for generating the visual code. For example, as described above, the computerized certification system 2 or its processing unit 20, respectively, generates transformed diamond reference characteristics by applying a cryptographic function to the diamond reference characteristics, particularly a cryptographic hash function, and includes the transformed diamond reference characteristics, particularly the cryptographic hash value of the diamond reference characteristics ("reference hash"), in the visual code.

In step S14, the computerized certification system 2 or its processing unit 20, respectively, controls the beam writing system 22 to write the visual code in or on the diamond 1.

Figure 10, illustrates an alternative sequence of the steps S11-S14 for certifying the diamond 1 in step S1 during the certification phase. Specifically, steps S13 and S14, for generating and writing the visual code in or on the diamond 1, are executed prior to steps S11 and S12, for generating the diamond reference characteristics and linking the diamond reference characteristics to the diamond 1. Essentially, with this alternative sequence, the physical parameters of the diamond 1 are measured after the visual code has been written in or on the diamond 1. Thereby, according to the sequence of Figure 10, the diamond reference characteristics reflect the visual code written in or on the diamond 1, however, the visual code does not include the diamond reference characteristics, as would be possible in the sequence according to Figure 9.

Figure 11, illustrates a combined embodiment of the sequences of Figures 9 and 10 for certifying the diamond 1 in step S1 during the certification phase. Specifically, in steps S11 and S12, a first set of the diamond reference characteristics are generated and linked to the diamond 1. In steps S13 and S14, using the first set of the diamond reference characteristics, the visual code is generated and written in or on the diamond 1. In additional step S11*, the computerized certification system 2 generates a second set of diamond reference characteristics of the diamond 1. The second set of the reference characteristics of the diamond 1 are generated as described above with reference to Figures 9 or 10. In additional step S12*, the computerized certification system 2 links the second set of diamond reference characteristics to the diamond 1. The second set of the reference characteristics are linked to the diamond 1 as described above with reference to Figures 9 or 10. Essentially, with this combined embodiment, a first set of physical parameters of the diamond 1 is measured before the visual code has been written in or on the diamond 1 and a second set of physical parameters of the diamond 1 is measured after the visual code has been written in or on the diamond 1. Thereby, the first set of the diamond reference characteristics are reflected in the visual code written in or on the diamond 1, whereas the second set of the diamond reference characteristics reflect the visual code written in or on the diamond 1.

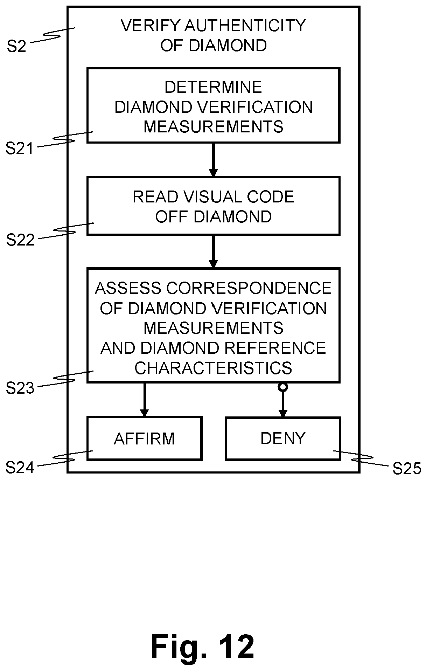

As illustrated in Figure 12, for authenticating the diamond 1 in step S2 during an authentication phase, in step S21, the computerized authentication system 3 determines diamond verification measurements of the diamond 1. More specifically, the processing unit 30 of the computerized authentication system 3 captures measurements of one or more physical characteristics of the diamond 1. For example, the processing unit 30 of the computerized authentication system 3 controls the sensor system 31 of the computerized authentication system 3 to measure the one or more physical characteristics of the diamond 1. Alternatively, the processing unit 30 of the computerized authentication system 3 receives the measurements of one or more physical characteristics of the diamond 1 via a user interface from a user who has used the sensor system 31 to measure the one or more physical characteristics of the diamond 1.

As described above, examples of the measured physical characteristics of the diamond 1 include the weight of the diamond 1, the color spectrum of the diamond 1, the chemical composition of the diamond 1, one or more images of the diamond 1, measurements and/or images of markings in or on the diamond 1, measurements and/or images of inclusions in the diamond 1, and/or three-dimensional topographies of the diamond 1, tomographic data of inclusions and/or markings and their locations in the diamond 1, and/or a hologram. In an embodiment, the measured physical characteristics of the diamond 1 further include the refraction index and/or the conductance of the diamond 1. As described above in connection with step S11, in an embodiment, the diamond reference characteristics include a secret diamond code; accordingly, generating the verification measurements in step S21 further comprises the computerized authentication system requesting and receiving via a user interface from a user the secret diamond code and including the secret diamond code received from the user in the diamond verification measurements.

In step S22, the visual code written in or on the diamond 1 is captured in the computerized authentication system 3. In an embodiment, the computerized authentication system 3 reads the visual code written in or on the diamond 1. More specifically, the processing unit 30 of the computerized authentication system 3 controls the sensor system 31 of the computerized authentication system 3 to read the visual code off the diamond 1. For example, the processing unit 30 of the computerized authentication system 3 controls the visual code reader of the sensor system 31 to read the visual code off the diamond 1. Alternatively, the computerized authentication system 3 receives the visual code via a user interface from a user who has read the visual code off the diamond 1.

The person skilled in the art will understand that the order of the sequence of step S21 and step S22 could be reversed without any change in the outcome.

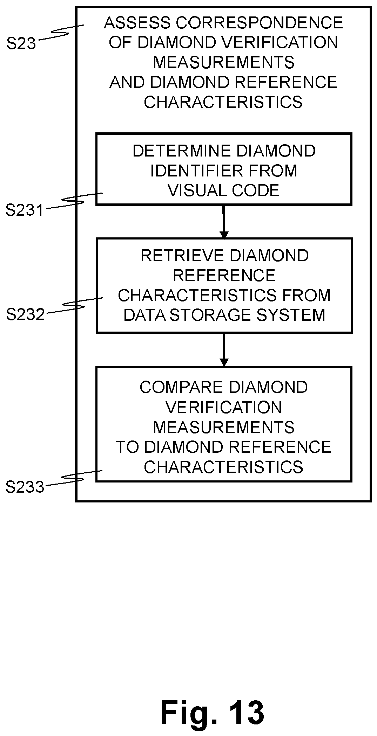

In step S23, the computerized authentication system 3 assesses the correspondence of the diamond verification measurements, determined for the diamond 1 in step S21, and the diamond reference characteristics of the diamond 1. Depending on the outcome of this assessment, the computerized authentication system 3 or its processing unit 30, respectively, affirms the authenticity of the diamond 1 in step S24, in case there is a positive correspondence of the diamond verification measurements and the diamond reference characteristics (i.e. the diamond verification measurements and the diamond reference characteristics match within a defined tolerance), or denies the authenticity of the diamond 1 in step S25, in case there is not a positive correspondence of the diamond verification measurements and the diamond reference characteristics (i.e. the diamond verification measurements and the diamond reference characteristics do not match within the defined tolerance). Various embodiments of step S23 for assessing this correspondence will be explained below with reference to Figures 13-18.

In step S24, the computerized authentication system 3 or its processing unit 30, respectively, affirms the authenticity of the diamond 1 by generating an authentication message. The computerized authentication system 3 or its processing unit 30, respectively, communicates the authentication message to the user via a user interface of the computerized authentication system 3, e.g. a display and/or a speaker. In an embodiment, the authentication message includes a digital signature, which is verifiable by a trusted third party, for example using Public Key Infrastructure (PKI). In an embodiment, the authentication message includes the material reference code or source information described above in connection with step S12. Accordingly, upon positive affirmation of the authenticity of the diamond 1, the computerized authentication system 3 or its processing unit 30, respectively, may render for the user, on the user interface of the computerized authentication system 3, at least some of the multimedia information related to the source and/or material used for producing the diamond 1.

In the embodiment or configuration of Figure 13, in step S231, the computerized authentication system 3 or its processing unit 30, respectively, determines the diamond identifier of the diamond 1 from the visual code captured from the diamond 1. In step S232, the computerized authentication system 3 or its processing unit 30, respectively, retrieves the diamond reference characteristics for the diamond 1 from a data storage system 200', using the diamond identifier of the diamond 1. In an embodiment, addressing information for the data storage system 200' is determined from the visual code captured from the diamond 1. In step S233, the computerized authentication system 3 or its processing unit 30, respectively, determines the authenticity of the diamond 1 by comparing the diamond verification measurements of the diamond 1 to the diamond reference characteristics of the diamond 1.

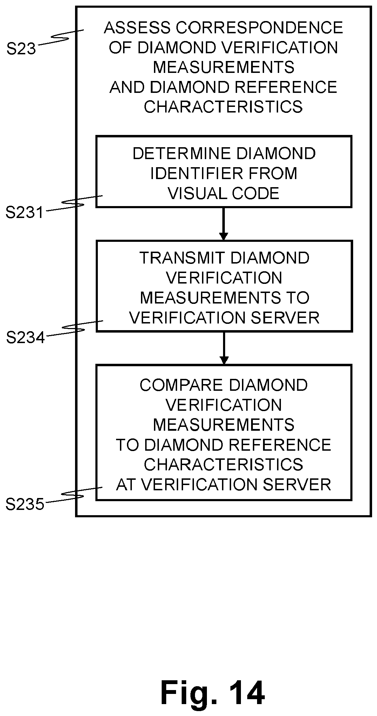

In the embodiment or configuration of Figure 14, in step S231, the computerized authentication system 3 or its processing unit 30, respectively, determines the diamond identifier of the diamond 1 from the visual code captured from the diamond 1. In step S234, the computerized authentication system 3 or its processing unit 30, respectively, transmits the diamond identifier of the diamond 1 and the diamond verification measurements determined for the diamond 1 to the verification server 4 of the computerized authentication system 3. In an embodiment, addressing information for the verification server 4 is determined from the visual code captured from the diamond 1. In step S235, the verification server 4 of the computerized authentication system 3 determines the authenticity of the diamond 1 by comparing the received diamond verification measurements to the diamond reference characteristics assigned to the received diamond identifier of the diamond 1 in the data storage system 200'.

In the embodiment or configuration of Figure 15, in step S236, the computerized authentication system 3 or its processing unit 30, respectively, determines the diamond reference characteristics from the visual code captured from the diamond 1. In step S237, the computerized authentication system 3 or its processing unit 30, respectively, determines the authenticity of the diamond 1 by comparing the diamond verification measurements of the diamond 1 to the diamond reference characteristics determined from the visual code.

In the embodiment or configuration of Figure 16, in step S238, the computerized authentication system 3 or its processing unit 30, respectively, generates transformed diamond verification measurements. For example, the computerized authentication system 3 or its processing unit 30, respectively, generates the transformed diamond verification measurements by applying a cryptographic function to the diamond verification measurements of the diamond 1, particularly a cryptographic hash function. As described above, in an embodiment, generating the transformed diamond verification measurements ("verification hash") comprises a discretization of the diamond verification measurements of the diamond 1 prior to applying the cryptographic function, specifically, the cryptographic hash function.

In step S239, the computerized authentication system 3 or its processing unit 30, respectively, determines transformed diamond reference characteristics ("reference hash") from the visual code captured from the diamond 1.

In step S240, the computerized authentication system 3 or its processing unit 30, respectively, determines the authenticity of the diamond 1 by comparing the transformed diamond verification measurements ("verification hash") of the diamond 1 to the transformed diamond reference characteristics ("reference hash") determined from the visual code.

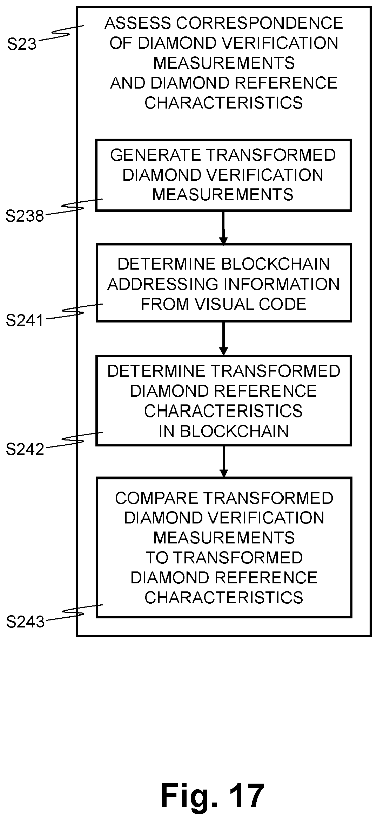

In the embodiment or configuration of Figure 17, in step S238, the computerized authentication system 3 or its processing unit 30, respectively, generates transformed diamond verification measurements, as described above with reference to Figure 16. In step S241, the computerized authentication system 3 or its processing unit 30, respectively, determines Blockchain addressing information for the Blockchain data storage system 200', e.g. a Blockchain account address or a Blockchain transaction identifier of a Blockchain record, from the visual code captured from the diamond 1. In step S242, the computerized authentication system 3 or its processing unit 30, respectively, determines transformed diamond reference characteristics from the Blockchain data storage system 200', using the Blockchain addressing information. In step S243, the computerized authentication system 3 or its processing unit 30, respectively, determines the authenticity of the diamond 1 by comparing the transformed diamond verification measurements ("verification hash") of the diamond 1 to the transformed diamond reference characteristics ("reference hash") determined in the Blockchain data storage system 200'.

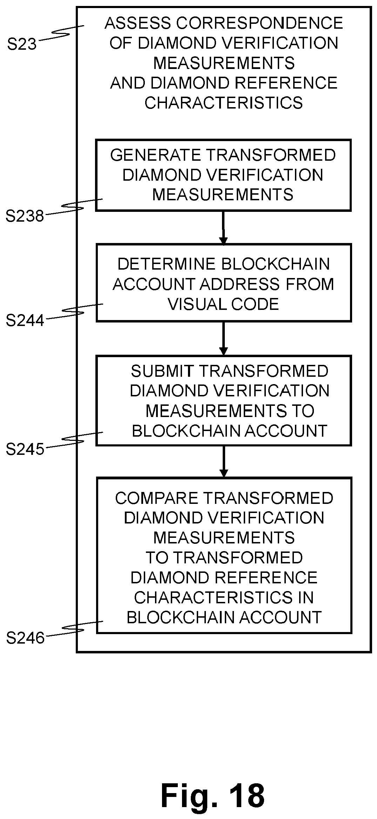

In the embodiment or configuration of Figure 18, in step S238, the computerized authentication system 3 or its processing unit 30, respectively, generates transformed diamond verification measurements, as described above with reference to Figures 16 and 17. In step S244, the computerized authentication system 3 or its processing unit 30, respectively, determines a Blockchain account address for the Blockchain data storage system 200', from the visual code captured from the diamond 1. In step S245, the computerized authentication system 3 or its processing unit 30, respectively, submits the transformed diamond verification measurements to the Blockchain account of the Blockchain data storage system 200', using the Blockchain account address. In step S246, the authenticity of the diamond 1 is determined by executing the code of a smart contract of the Blockchain account to compare the transformed diamond verification measurements ("verification hash") of the diamond 1 to the transformed diamond reference characteristics ("reference hash") stored in the Blockchain data storage system 200', particularly in the addressed account of the Blockchain data storage system 200'. For example, the code of the smart contract is executed by the computerized authentication system 3 or its processing unit 30, respectively, implementing a node of the Blockchain data storage system 200'.

It should be noted that, in the description, the sequence of the steps has been presented in a specific order, one skilled in the art will understand, however, that the order of at least some of the steps could be altered, without deviating from the scope of the disclosure.

## Claims

1. A method of determining authenticity of a manufactured diamond (1), comprising:
generating (S11) diamond reference characteristics by a computerized certification system (2) capturing measurements of one or more physical characteristics of the diamond (1) during a certification phase;
generating, by the computerized certification system (2), a hash of the diamond reference characteristics by applying a cryptographic hash function to the diamond reference characteristics;
generating (S13), by the computerized certification system (2), a visual code for the diamond (1), using the hash of the diamond reference characteristics;
writing (S14) the visual code in or on the diamond (1), using a beam writing system (22); and
verifying (S2) the authenticity of the diamond (1) by:
generating (S21) diamond verification measurements by a computerized authentication system (3) capturing measurements of the one or more physical characteristics of the diamond (1) during an authentication phase;
generating a hash of the diamond verification measurements by the computerized authentication system (3) applying the cryptographic hash function to the diamond verification measurements;
capturing (S22), in the computerized authentication system (3), the visual code of the diamond (1);
comparing, by the computerized authentication system (3), the hash of the diamond verification measurements with the hash of the diamond reference characteristic, using the visual code captured from the diamond (1); and
affirming (S24) or denying (S25) the authenticity of the diamond (1), by the computerized authentication system (3), depending on the comparing.

2. The method of claim 1, wherein the method further comprises the computerized certification system (2) storing the diamond reference characteristics assigned to a unique diamond identifier in a data storage system (200); the computerized certification system (2) including the unique diamond identifier in the visual code; the computerized authentication system (3) determining the diamond identifier from the visual code; and the computerized authentication system (3) comparing (S233) the diamond verification measurements to at least some of the diamond reference characteristics, stored and assigned in the data storage system (200) to the diamond identifier.

3. The method of claim 2, wherein comparing (S233) the diamond verification measurements with the diamond reference characteristics comprises at least one of: retrieving (S232) at least some of the diamond reference characteristics via a network (5) from the data storage system (200), or transmitting (S234) at least some of the diamond verification measurements and the diamond identifier via the network (5) to a verification server (4).

4. The method of claim 3, wherein generating (S13) the visual code comprises including in the visual code addressing information of at least one of: the data storage system (200) or the verification server (4); and comparing (S233) the diamond verification measurements with the diamond reference characteristics comprises using the addressing information included in the visual code for at least one of: retrieving (S232) at least some of the diamond reference characteristics from the data storage system (200) or transmitting (S234) at least some of the diamond verification measurements and the diamond identifier to the verification server (4).

5. The method of one of claims 1 to 4, wherein the method further comprises the computerized certification system (2) storing the hash of the diamond reference characteristics in a Blockchain data storage system (200); and the computerized authentication system (3) comparing (S240) the hash of the diamond verification measurements with the hash of the diamond reference characteristics stored in the Blockchain data storage system.

6. The method of one of claims 1 to 5, further comprises generating markings in or on the diamond (1), using a beam writing system (22).

7. The method of claim 6, wherein generating markings in or on the diamond (1) comprises at least one of: varying an energy level of the beam writing system (22), varying a degree of overlap of pulses of the beam writing system (22), varying a projection direction of a beam of the beam writing system (22), or varying a focal position of the beam of the beam writing system (22).

8. The method of one of claims 1 to 7, further comprises generating markings on the diamond, using at least one of: potassium nitrate, plasma etching in combination with masking materials, gas phase etching in dry oxygen, gas phase etching in a mixture of oxygen and water vapor, or liquid etching in molten potassium nitrate.

9. The method of one of claims 1 to 8, wherein measuring the one or more physical characteristics of the diamond (1) comprises at least one of: measuring a weight of the diamond (1), measuring a three-dimensional geometric shape of the diamond (1), measuring a color spectrum of the diamond (1), measuring a chemical composition of the diamond (1), measuring markings in or on the diamond (1), measuring inclusions in the diamond (1), or measuring a hologram in the diamond (1).

10. The method of one of claims 1 to 9, wherein generating (S11) the diamond reference characteristics comprises the computerized certification system (2) including a secret diamond code in the diamond reference characteristics; and generating (S21) the verification measurements comprises the computerized authentication system (3) receiving from a user the secret diamond code and including the secret diamond code received from the user in the diamond verification measurements.

11. The method of one of claims 1 to 10, wherein generating (S13) the visual code comprises generating a QR-code, and writing (S14) the visual code in or on the diamond (1) comprises writing the QR-code in or on the diamond (1) using the beam writing system (22).

12. The method of one of claims 1 to 11, wherein affirming (S24) the authenticity of the diamond (1) comprises generating an authentication message, the authentication message including a digital signature, which is verifiable by a trusted third party.

13. The method of claim 12, wherein generating (S13) the visual code comprises including in the visual code addressing information of the trusted third part.

14. A computerized authentication system (3) for verifying authenticity of a manufactured diamond (1), the manufactured diamond (1) comprising a visual code, the computerized authentication system (3) comprising a processing unit (30) and a sensor system (31) connected to the processing unit (30), wherein the processing unit (30) is configured to:
capture one or more physical characteristics of the diamond (1) measured by the sensor system (31) during an authentication phase,
generate diamond verification measurements using the one or more physical characteristics of the diamond (1) measured during the authentication phase,
generate a hash of the diamond verification measurements by applying a cryptographic hash function to the diamond verification measurements;
capture the visual code of the diamond (1),
using the visual code captured from the diamond to compare the hash of the diamond verification measurements with a hash of diamond reference characteristic, generated by a computerized certification system (2) from one or more physical characteristics of the diamond (1) during a certification phase, and
affirm or deny the authenticity of the diamond (1) depending on the comparison.

15. A computer program product comprising a non-transitory computer readable medium having stored thereon computer program code configured to control a processing unit (30) of a computerized authentication system (3) to verify authenticity of a manufactured diamond (1), the manufactured diamond (1) comprising a visual code, by performing the following steps:
capturing one or more physical characteristics of the diamond (1) measured by a sensor system (31) of the computerized authentication system (3) during an authentication phase,
generating diamond verification measurements using the one or more physical characteristics of the diamond (1) measured during the authentication phase,
generating a hash of the diamond verification measurements by applying a cryptographic hash function to the diamond verification measurements;
capturing the visual code of the diamond (1),
using the visual code captured from the diamond to compare the hash of the diamond verification measurements and a hash of diamond reference characteristic, generated by a computerized certification system (2) from one or more physical characteristics of the diamond (1) during a certification phase, and
affirming or denying the authenticity of the diamond (1) depending on the comparison.
